(19) Europäisches Patentamt

European Patent Office

Office européen des brevets

(11) Veröffentlichungsnummer: **0 310 984 B1**

(12) **EUROPÄISCHE PATENTSCHRIFT**

(45) Veröffentlichungstag der Patentschrift: **20.05.92**

(21) Anmeldenummer: **88116301.8**

(22) Anmeldetag: **01.10.88**

(51) Int. Cl.⁵: **A61K 9/50, B01J 13/00**

(54) **Verfahren und Vorrichtung zur Herstellung Wässriger Kolloide.**

(30) Priorität: **05.10.87 DE 3733652**

(43) Veröffentlichungstag der Anmeldung:
**12.04.89 Patentblatt 89/15**

(45) Bekanntmachung des Hinweises auf die
Patenterteilung:
**20.05.92 Patentblatt 92/21**

(84) Benannte Vertragsstaaten:
**AT BE CH DE FR GB IT LI LU NL SE**

(56) Entgegenhaltungen:
**EP-A- 0 072 703**
**WO-A-86/01714**
**US-A- 3 015 128**

(73) Patentinhaber: **ABNOBA HEILMITTEL GMBH**
**Güterstrasse 53**
**W-7530 Pforzheim(DE)**

(72) Erfinder: **Koehler, Reinhard, Dr.**
**Obere Bachgasse 70**
**W-7532 Niefern-Öschelbronn(DE)**
Erfinder: **Schäfer, Gerhard**
**Ahornstrasse 3**
**W-7532 Niefern-Öschelbronn(DE)**

(74) Vertreter: **Buchner, Otto, Dr. et al**
**Patentanwälte Dipl.-Ing. Klaus Westphal Dr.**
**rer. nat. Bernd Mussgnug Dr. rer. nat. Otto**
**Buchner Flossmannstrasse 30a**
**W-8000 München 60(DE)**

## Beschreibung

Die Erfindung betrifft ein Verfahren zur Herstellung wässriger Kolloide nach dem Oberbegriff des Anspruchs 1 sowie eine Vorrichtung zur Durchführung dieses Verfahrens.

Bei bekannten Verfahren dieser Art werden in Spaltströmungen oder durch Rührwerke stark vergrößerte Grenzflächen zwischen den Mischungskomponenten erzeugt. Beispielsweise kann bei der Emulgierung von Wasser und Öl mit einem Emulgator, wenn die einzelnen Bestandteil das richtige Mengenverhältnis haben, eine mehr oder weniger feine, stabile Emulsion erzeugt werden.

Ferner sind Verfahren bekannt, amphiphile Lipide synthetischen oder biologischen Ursprungs in die Struktur einer Doppellamelle zu bringen, die beiderseits von wässrigem Milieu begrenzt wird. Auf dieser Grundlage kann im einfachsten Fall durch vorübergehende Zugabe eines Detergens in hoher Konzentration eine Suspension von kugelförmigen Lamellen, sogenannten Liposomen, erzeugt werden. Durch geeignetes Vorgehen kann dabei sowohl das Detergens, allerdings nur bis auf einen unter Umständen sehr störenden Rest, wieder entfernt als auch der Einschluß von bestimmten Stoffen, z.B. Arzneisubstanzen, in das innere wässrige Milieu der Liposomen bewirkt werden. Das äußere wässrige Milieu der Liposomen kann durch Waschen und Filtrieren von dem zugegebenen Stoff befreit und durch weitere Zusätze beeinflußt werden. Eine Suspension solcher Liposomen stellt, im Vergleich zu einer einfachen Emulsion z.B. vom Öl/Wasser-Typ, ein Kolloid mit höherem Ordnungsgrad dar.

Die Herstellung solcher wässrigen Kolloide wurde in den letzten 20 Jahren vor allem auf chemischem Wege bei der Membransynthese erreicht. Es wäre wünschenswert, sie auch auf physikalischem Wege zu erreichen. Das bekannte Dispergieren und Emulgieren könnte, da die Anforderungen hier geringer sind als bei der Membransynthese, in gleicher Weise und in sehr kurzer Zeit betrieben werden. Im Falle des Dispergierens und Emulgierens kommen sowohl wässrige als auch alle anderen, z.B. ölige Flüssigkeiten in Betracht.

Der Erfindung liegt die Aufgabe zugrunde, ein Verfahren und eine Vorrichtung der eingangs genannten Art so auszubilden, daß Kolloide mit dem oben erklärten höheren Ordnungsgrad erzeugt werden können, wobei die chemischen Beeinträchtigungen bisher bekannter Verfahren vermieden werden sollen.

Diese Aufgabe wird erfindungsgemäß durch die kennzeichnenden Merkmale des Anspruchs 1 bzw. die Merkmale des Anspruchs 5 gelöst. Dabei erweist sich der gemäß Anspruch 1 zur Mischung verwendete, rotierende Flüssigkeitsring als besonders vorteilhaft, weil die als Rieselfilm und Tropfennebel bewegten Mischungskomponenten im Moment der Mischung sehr schnell von dem umgebenden Gas durch Zentrifugierwirkung getrennt werden. Einstrudeln von Gasbläschen, unkontrolliertes Spreiten und Schäumen nach der Mischung können vollkommen ausgeschlossen werden.

Bei dem erfindungsgemäßen Verahren erfolgen Oberflächenbildung und Spreiten für jede der beiden Ausgangsflüssigkeiten getrennt, und zwar vor der Mischung einerseits durch einen Rieselfilm auf rotierender Scheibe und andererseits durch einen Tropfennebel. Dagegen spreiten die oberflächenaktiven Bestandteile (z.B. Emulgatoren) bei den bisherigen Emulgierverfahren während des Mischens oder nach Ruhenlassen der Mischung. Beim erfindungsgemäßen Verfahren werden Rieselfilm und Tropfennebel erst nach vollständigem Spreiten in Form einer hier sogenannten Oberflächenmischung zur Doppellamelle zusammengeführt; anschließend werden die dabei entstehenden lamellaren Elemente (Liposomen bzw. Membranvesikel) durch die von dem rotierenden Flüssigkeitsring gebildete Ringströmung in das wässrige Milieu der Pufferlösung eingebettet. Auf diesem Wege gelingt es, ein stabiles Kolloid höherer Ordnung zu erzeugen und eine Membransynthese auf physikalischem Wege zu erreichen.

Werden Suspensionen, welche Vesikel mit Lipid-Doppellamellen oder Lipid-Protein-Membranen enthalten, an den Oberflächen der zwei Flüssigkeitsströme zum Spreiten gebracht, dann geht die Doppelstruktur der Vesikelmembranen in die Einfachstruktur ihrer Membranhälften über. Die Synthese zur Membrandoppelstruktur schließt sich an, indem die beiden Flüssigkeitsströme als Rieselfilm und Tropfennebel zur Berührung gebracht werden.

Zwei gleiche Suspensionen mit Vesikeln aus symmetrischen Membranen würden auf diese Weise eine gleichartige Vesikelsuspension, d.h. mit wieder symmetrischer Membran ergeben. Eigentliches Ziel des Verfahrens ist aber die Synthese asymmetrischer Membranstrukturen dadurch, daß von zwei verschiedenen Suspensionen ausgegangen wird.

Die Unteransprüche 2 bis 4 betreffen vorteilhafte Ausführungsformen des erfindungsgemäßen Verfahrens.

Anspruch 2 betrifft eine zweckmäßige Ausführungsform des Verfahrens, bei dem die Volumenströme von Rieselfilm und Tropfennebel so aufeinander abgestimmt werden, daß die je Sekunde gebildeten Oberflächen gleich werden, und die Suspensionen in solcher Konzentraton zufließen, daß ein Filmdruck von 5 bis 25 dyn/cm ($5 \cdot 10^{-5}$ - $2,5 \cdot 10^{-4}$ N/cm) an den in die Mischung eingehenden Oberflächen entsteht.

Insbesondere betrifft Anspruch 3 eine zweckmäßige Ausgestaltung des Verfahrens, bei der die Drehbewegung des rotierenden Flüssigkeitsringes, ausgehend von einer hohen Anfangsdrehzahl, langsam verringert wird. Durch die Verzögerung wird eine Ringströmung mit starker, auf Corioliskräften beruhender Sekundärströmung bewirkt. Die Ringströmung begünstigt das Einbetten der Vesikel in dei wässrige Pufferlösung durch den im rotierenden Ring vorhandenen hohen hydrostatischen Druck und verhindert gleichzeitig durch die an den Wänden auftretenden Scherschichten ein Sedimentieren und Koagulieren der Kolloidelemente. Wenn dem Rieselfilm, der sich auf dem gleichen Rotor wie der Flüssigkeitsring befindet, umgekehrt proportional zur Drehzahl mehr Flüssigkeit zugeführt wird, dann kann die Wirkung der abnehmenden Drehzahl auf den Rieselfilm kompensiert wreden. Die je Sekunde gebildete Oberfläche bleibt dann konstant.

Besonders vorteilhaft läßt sich das erfindungsgemäße Verfahren auf die Kolloidbildung mit pflanzlichen Preßsäften, insbesondere Preßsäften von Viscum album L. gleicher oder verschiedener Herkunft anwenden.

Die erfindungsgemäße Vorrichtung gemäß Anspruch 5 ist zur Erzeugung der beiden erforderlichen großen Oberflächen und zur Erzeugung der erwähnten Ringströmung geeignet. Allerdings muß der fliegend gelagerte Rotor zur Aufnahme des Flüssigkeitsrings gut stabilisiert werden.

Die Unteransprüche 6 bis 20 betreffen vorteilhafte Ausgestaltungen der erfindungsgemäßen Vorrichtung nach Anspruch 5.

Für die Stabilisierung des Rotors werden bei einer besonders zweckmäßigen Ausführungsform gemäß Anspruch 7 bis 9 ein axial verschiebbares Fanglager, eine Ölringdämpfung und eine äußere Dämpfung verwendet. Dadurch kann der gesamte Drehzahlbereich von Instabilitäten des Rotors freigehalten werden, welche auf dynamischen Unwuchten der Flüssigkeitsfüllung im Rotor beruhen und wie bekannt etwa zwischen dem halben und doppelten Betrag einer jeden kritischen Drehzahl liegen.

Das Spreiten der ersten Suspension auf dem Rieselfilm bis zum notwendigen Filmdruck kann gemäß Anspruch 4 und 12 zweckmäßig durch Vorschalten eines Rieselvorlaufs in Form einer Filterkerze erreicht werden.

Der Tropfennebel wird in vorteilhafter Art durch eine rotierende Sprühscheibe gemäß Anspruch 15 erzeugt, wobei eine nur geringe Scherbeanspruchung der empfindlichen Lipide und Proteine auftritt und trotzdem feine Tropfen einheitlicher Größe entstehen.

Das Spreiten der zweiten Suspension auf dem Tropfennebel kann ähnlich wie beim Rieselfilm durch Vorschalten einer Doppeldüse gemäß Anspruch 19 beschleunigt werden.

Die Ansprüche 14 und 16 bis 18 betreffen Einbauten zum Abbremsen der Sprühscheibe abgeschleuderten Tröpfchen und deren Führung durch einen Gasstrom zum Mischungsort.

Anhand der Figuren wird das erfindungsgemäße Verfahren sowie eine Vorrichtung zur Durchführung des erfindungsgemäßen Verfahrens näher erläutert, und zwar an einem für die Membransynthese geeigneten Beispiel. Es zeigt

Fig.1 eine teilweise geschnittene Schrägansicht des auf dem Abtriebswellenstumpf sitzenden Rotors von oben,

Fig.2 einen schematischen Vertikalschnitt durch den Rotor und die Vorrichtung zum Antrieb und zur Lagerung bzw. Stabilisierung des Rotorsystems,

Fig.2a die durch den Kreis IIa in Fig.2 eingerahmte Einzelheit in vergrößertem Maßstab,

Fig.3 eine graphische Darstellung, in der die Stabilität der radialen Rotorschwerpunktbewegung gegen die Rotordrehzahl aufgetragen ist,

Fig.4 einen schematischen Vertikalschnitt eines Rieselvorlaufs, welcher zum Spreiten der ersten Suspension und deren Zufluß zum Rieselfilm eingesetzt werden kann.

Fig.5 einen schematischen Vertikalschnitt durch einen Grobvakuumkessel mit einer Vorrichtung zur Erzeugung einer drehfreien Gasströmung, dem Rieselvorlauf über der Rotormitte und einer Sprüheinrichtung,

Fig.6 das wellige Randprofil einer Sprühscheibe in der Abwicklung ihrer Mantelfläche, wobei der Vertikalmaßstab vierfach überhöht ist, und

Fig.7 einen Ausschnitt von Fig.5 mit der Sprühscheibe und einer Doppeldüse für den Zulauf der zweiten Suspension.

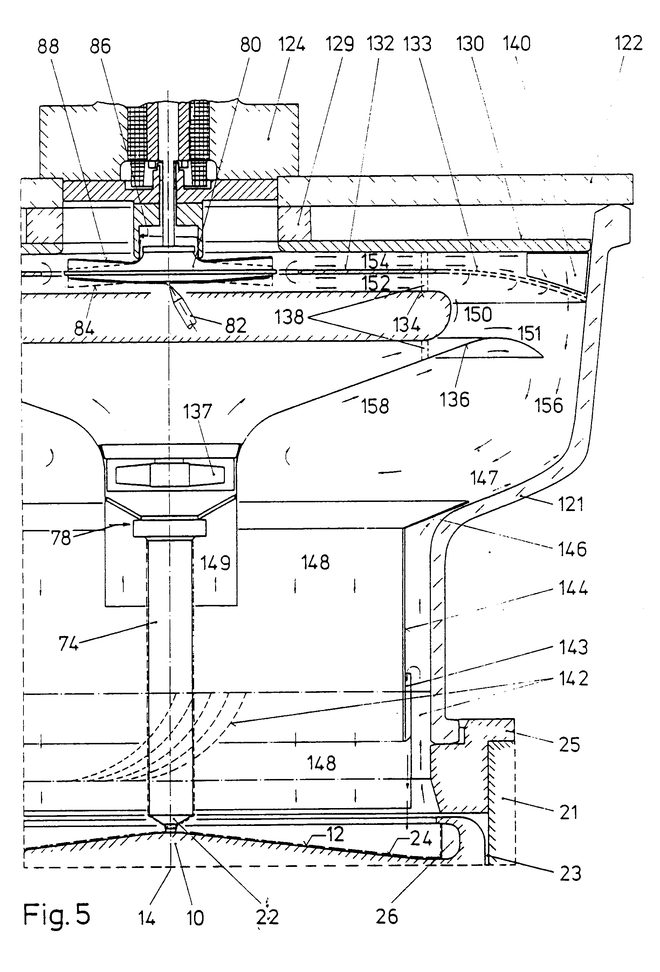

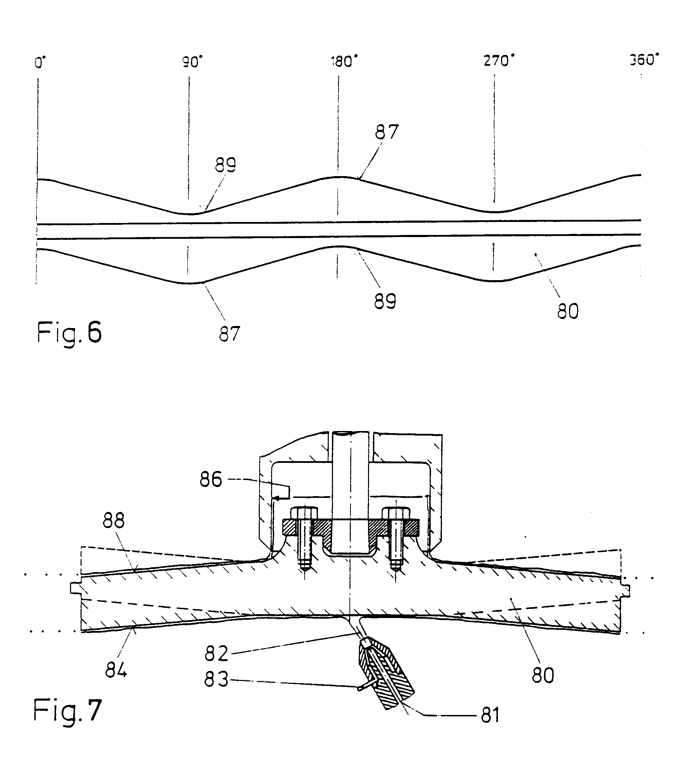

In Fig.5 und 7 sind die Profile der welligen Sprühscheibe aus zwei Schnittebenen übereinandergelegt: der in der Zeichenebene liegende Schnitt mit ausgezogenen Linien, der in 90°-Stellung liegende gestrichelt.

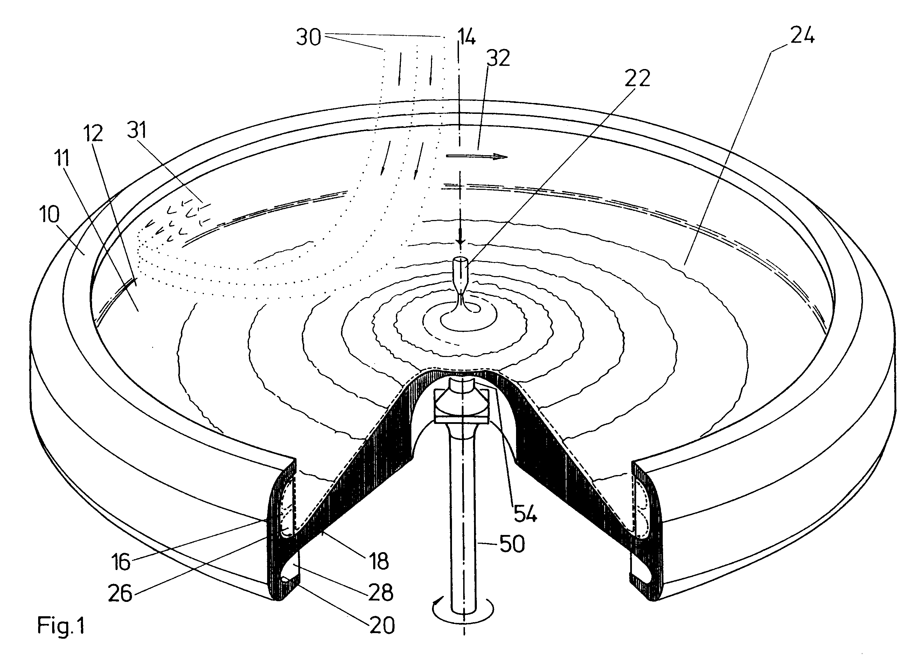

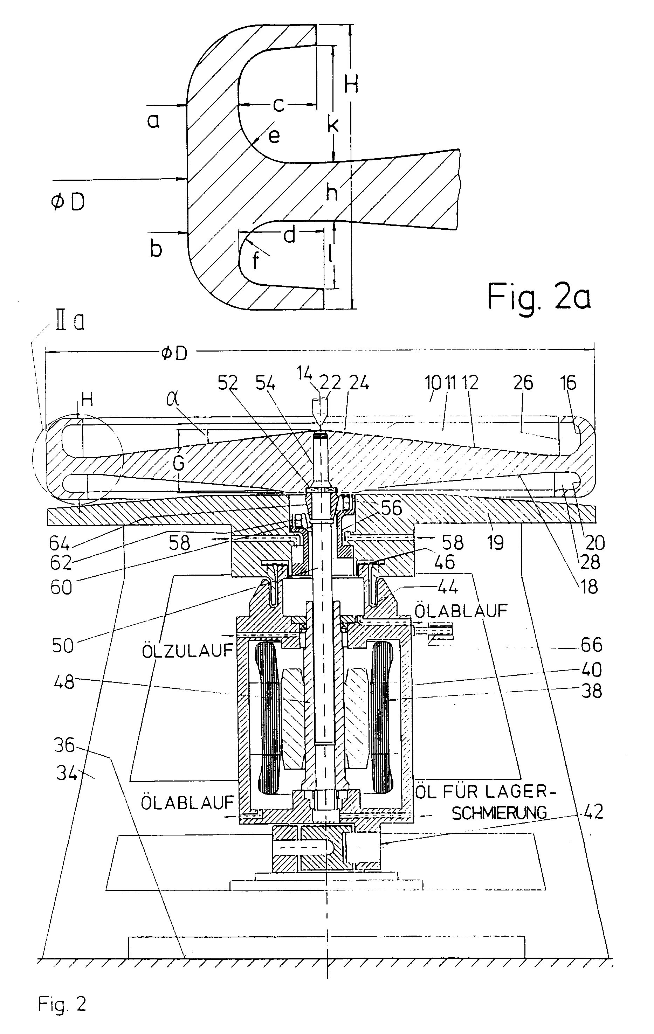

Die in den Fig.1, 2 und 2a dargestellte Vorrichtung weist einen allgemein mit 10 bezeichneten, im Querschnitt etwa schiebenförmigen Rotor auf, der an seiner Oberseite eine nach oben offene Schale 11 bildet, deren flacher Boden 12 in radialer Richtung von der Rotorachse 41 nach außen leicht nach unten geneigt ist. Am Umfang des Rotors 10 ist die Schale 11 nach oben und innen zur Bildung einer zur Rotorachse 14 hin offenen und um den ganzen Umfang verlaufenden Rinne 16 geformt. Eine ähnliche, jedoch nach unten offene und kleinere Schale 18 ist an der Unterseite des Rotors 10 ausgebildet, die in gleicher Weise mit einer um den Umfang laufenden und nach innen zur Rotorachse 14 hin offenen Rinne 20 versehen ist.

In kurzem Abstand oberhalb der Mitte des Bodens 12 ist eine in Fig.1, 2 nur schematisch angedeutete Zuflußdüse 22 für die erste Suspension angeordnet, die den Fig.1 angedeuteten Rieselfilm 24 auf dem Boden 12 bildet. Bei Drehung des Rotors 10 bewegt sich der Rieselfilm auf dem Boden 12 nach außen und wird in der Rinne 16 aufgefangen, wo er einen rotierenden Flüssigkeitsring 26 bildet.

Die untere Schale 18 wird vor dem Einbau des Rotors mit einem Blechdeckel in nicht näher gezeigter Weise abgedichtet und mit Öl hoher Zähigkeit gefüllt, das in der unteren Rinne 20 einen rotierenden Ölring mit frei verschieblicher Oberfläche bildet, wenn der Rotor hochgefahren wird.

Durch eine in Fig.5 gezeigte, in Fig. 1 jedoch weggelassene Sprüheinrichtung wird oberhalb der Schale 11 ein durch punktierte Linien angedeuteter Tropfennebel 30 aus der zweiten Suspension erzeugt, der sich gemäß den dort eingezeichneten Pfeilen nach unten bewegt und in Berührung mit dem Rieselfilm 24 auf dem rotierenden Flüssigkeitsring kommt. Fig.1 deutet dies schematisch an. Man hat sich aber vorzustellen, daß der Tropfennebel 30 in der Schale 11 allseitig radial nach außen bewegt und zum streifenden Aufprall 31 am rotierenden Flüssigkeitsring 26 gebracht wird. Durch diese verschiedenen Bewegungen der beiden Suspensionen erfolgt die Zusammenführung der beiden gebildeten Oberflächen und die oben erwähnte Bildung von Liposomen bzw. Membranvesikeln.

Wesentlich ist nun, daß zu Beginn des Verfahrens der Rotor auf seine Maximaldrehzahl, bei einer bevorzugten Ausführungsform 6000 bis 9000 U/min, gebracht wird, wobei die Rinne 16 mit einer Anfangsfüllung wässriger Pufferlösung von etwa 200 ml versehen wird. Dann wird die Drehzahl allmählich vermindert, während die beiden Suspensionen als Rieselfilm 24 und Tropfennebel 30 den Flüssigkeitsring 26 füllen und dieser die Ringströmung 32 ausbildet. Das Verfahren läuft dann bei deisem Ausführungsbeispiel während einer Dauer von ca. 3 min, bis die Rinne 16 randvoll mit der Mischung ist, einem Volumen von etwa 2200 ml entsprechend. Nach dem Abbremsen bis zum Stillstand des Rotors 10 wird die Mischung entnommen. Demgemäß ist das Verfahren bei dieser Ausführungsform diskontiuierlich. Bei Anwendung eines kontinuierlichen Durchflußprinzips müsste die über den Rand des Rotors ablaufende Flüssigkeit in Form sehr kleiner Tröpfchen absprühen. Erfahrungsgemäß wird dadurch ein beachtlicher Teil dieser Flüssigkeit in seiner Kolloidstruktur gestört und zum Ausflocken gebracht.

Der Rieselfilm 24 kann eine sehr geringe Dicke haben, beim erwähnten Ausführungsbeispiel weniger als $5\mu m$, wenn mit etwa 5ml/s dosiert wird.

Eine Grundschicht des Rieselfilms 24 wird durch wässrige Pufferlösung gebildet. Eine Deckschicht mit einem kleineren Anteil von etwa 40% am Rieselfilmzufluß wird von der der Oberflächenbildung auszusetzenden ersten Suspension, beispielsweise einem pflanzlichen Preßsaft, gebildet. Dieser wird auf dem Rieselfilm zum Spreiten gebracht, und zwar mit einem Filmdruck im Bereich von 5 bis 25 dyn/cm ($5 \cdot 10^{-5}$ - $2,5 \cdot 10^{-4}$ N/cm). Ähnlich wird bei der Zuführung der den Tropfennebel 30 bildenden zweiten Suspension verfahren. Dabei wird darauf geachtet, daß die mit den beiden Suspensionen je Zeiteinheit gebildeten Oberflächen gleich groß werden.

Für eine langsam spreitende Suspension wird alternativ zur Zuflußdüse 22 des Rieselfilms 24 in Fig.1, 2 ein Rieselvorlauf in Form einer Filterkerze eingesetzt. Denn der Rieselfilm 24 fließt so schnell über den Boden 12 zum Flüssigkeitsring 26, daß hier ein nur kleiner Teil einer langsam spreitenden Suspension den Phasenübergang zur Wasseroberfläche mitmachen würde.

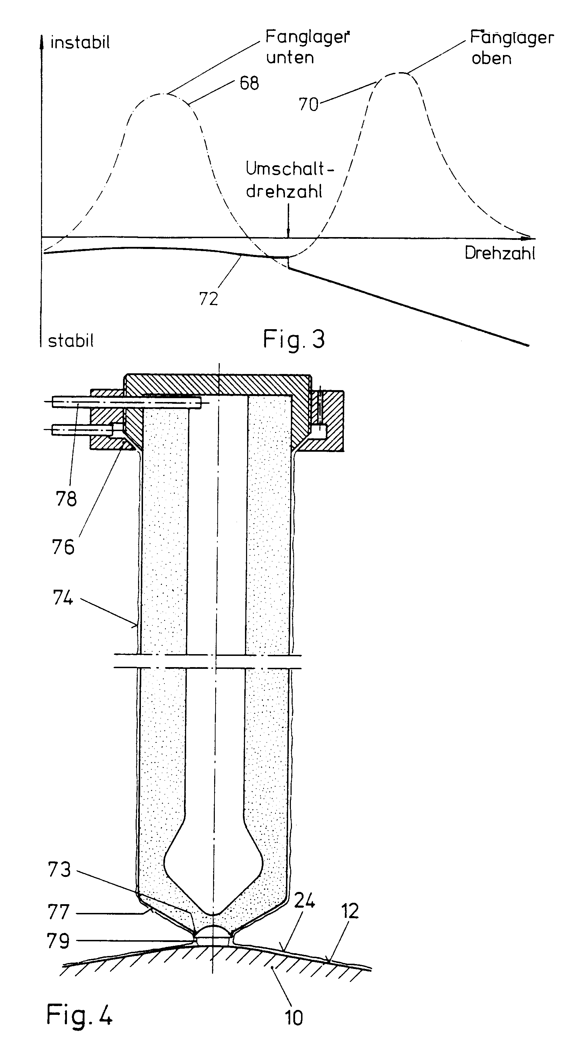

Fig.4 zeigt eine zweckmäßige Ausführung: Auf der dargestellten Filterkerze 74 strömt wässrige Pufferlösung gleichmäßig von oben innen 78 durch die poröse Wand zur Außenfläche und fließt dort als dünner Film langsam herab. Die erste Suspension wird durch den Ringspalt 76 oben außen gleichmäßig zugegeben, so daß deren oberflächenaktive Anteile allmählich an der Wasseroberfläche spreiten. Indem weiter unten stetig neue Pufferlösung von innen zufließt, nehmen die abwärtsgerichtete Oberflächengeschwindigkeit und die dem Spreiten zur Verfügung stehende Fläche zu. Am konischen Ende 77 schnürt sich der Flüssigkeitsfilm zusammen, wobei die Oberflächengeschwindigkeit bei dort abnehmender Wanddicke immer noch zunehmen oder konstant bleiben kann.

Der wenige mm messende Abstand zwischen der Schneide 73 am konischen Ende 77 und dem rotierenden Boden 12 kann von dem dort frei strömenden Flüssigkeitsfilm 79 überbrückt werden, der in den Rieselfilm 24 übergeht.

Die Rotordrehzahl wird während des Verfahrensablaufs, ausgehend von der Maximaldrehzahl, um ca. 10 bis 50% vermindert. Dabei wird der Zufluß an Pufferlösung für den Rieselfilm 24 so gesteuert, daß das Produkt aus dem Gesamtzufluß (etwa 60% Pufferlösung und 40% Suspension) und der Rotordrehzahl etwa konstant bleibt. Die Zuführung der Pufferlösungen und der Suspensionen zum Rieselfilm 24 und zum Tropfennebel 30 kann durch elektronisch gesteuerte Dosierkolben erfolgen, so daß die für die Mischung geeigneten Konzentrationen erhalten werden.

Die ganze Vorrichtung ist zweckmäßigerweise von einem in Fig.2 und 5 dargestellten Kessel 19, 21, 25 bzw. 121 umgeben, der unter einem Grobvakuum von etwa 50 mb steht.

Zur hohen Oberflächenbildung des Rieselfilms 24 ist eine genügend hohe Drehzahl des Rotors 10 erforderlich. Deshalb muß er aus Material hoher Festigkeit, bezogen auf die Materialdichte, bestehen. Auf die erreichbare Maximaldrehzahl hat die Formgebung des Rotors, mit den Materialdicken an den Rinnen 16 und 20 sowie den Krümmungsradien an diesen Stellen, Einfluß. Die Zahlenangaben für eine besonders zweckmäßige Ausführungsform des Rotors 10 sind in Anspruch 13 gegeben; der Durchmesser D des Rotors 10 beträgt bei diesem Ausführungsbeispiel 72 cm (Fig.2, 2a). Ebenso wird der Abtriebswellenstumpf 50 mit einem sehr kleinen Durchmesser von beispielsweise 18 mm ausgeführt, um den in der Paßbohrung 54 entstehenden Spitzenwert der Umfangsspannung abzumindern. Um Verschmutzungen durch Öl oder Fett unter allen Umständen zu vermeiden, wird der Rotor 10 mit seiner Paßbohrung 54 ohne Verschraubung auf das obere Ende 52 des Abtriebswellenstumpfes 50 aufgesetzt und durch sein Gewicht in der richtigen Lage gehalten.

Unterhalb des Rotors 10 ist ein allgemein mit 34 bezeichnetes und auf dem Fundament 36 aufruhendes Maschinengestell angeordnet. Im Maschinengestell 34 ist ein Elektromotor 38 in einem Pendelgehäuse 40 angeordnet. Das Pendelgehäuse 40 ist über ein Kardangelenk 42 auf dem Maschinengestell 34 abgestützt. Das Pendelgehäuse 40 ist über eine an sich bekannte Ölspalt-Dämpfungskammer 44 mit Membrandichtung 46 pendelnd im Maschinengestell 34 gelagert.

Der Motor 38 weist eine biegesteife Antriebswelle 48 großen Durchmessers auf, die einen Kurzschlußläufer trägt und die oberhalb des Motorgehäuses in einen biege- und torsionsweichen Abtriebswellenstumpf 50 übergeht. Das geeignet mit Kupplungsflächen versehene obere Ende 52 des Wellenstumpfes 50 sitzt in einer entsprechenden Paßbohrung 54 des Rotors und ist somit antriebsmäßig mit diesem verbunden.

Der Abtriebswellenstumpf 50 ist von einem vertikal verstellbaren Fanglager 56 umgeben, das zwischen der in der linken Hälfte der Fig.2 dargestellten unteren und der in der rechten Hälfte der Fig.2 dargestellten oberen Position umschaltbar ist. Zur Verstellung des Fanglagers 56 wird Hydrauliköl gemäß den Doppelpfeilen 58 ein- bzw. ausgeleitet. Das Fanglager 56 stützt sich in der oberen Position mit minimalem Spiel radial gegen den am Maschinengestell 34 verschraubten Kesselboden 19 ab und enthält einen über das Kugellager 60 gelagerten Laufring 62, der sich mit seiner Inneseite an einen nahe dem oberen Ende des Abtriebswellenstumpfes 50 befestigten Konus 64 flächig anlegt.

Gleichzeitig mit dem Anlegen des Fanglagers 56 an den Antriebswellenstumpf 50 muß auch das Pendelgehäuse 40 des Motors festgelegt werden. Dies erfolgt bei der dargestellten Ausführungsform durch vier jeweils um 90 Grad winkelversetzte Spindeln 66, von denen in Fig.2 nur eine dargestellt ist. Durch Anfahren der Spindelenden an das Pendelgehäuse 40 wird das letztere festgelegt.

Anhand der Fig.2, 3 und 4 wird die Wirkung des Fanglagers auf die Stabilität der Rotorbewegung näher erläutert. Als Stabilität wird derjenige Zustand des Rotor-Welle-Lagersystems bezeichnet, in dem alle radialen Eigenschwingungen gedämpft sind. Diese werden bei Rotation durch Unwuchten der festen oder flüssigen Massen und durch Reibungskräfte in den Lagern oder im Gasspalt 23 angeregt. Instabilität bedeutet dann also Anfachung der Eigenschwingungen. Besonders die vier Eigenschwingungen der im mitrotierenden Flüssigkeitsring 26 und Ölring 28 gleich oder gegensinnig zur Rotation umlaufenden Wellen können in bestimmten, sehr breiten Drehzahlbereichen schnell zunehmende Unwuchten erzeugen.

Im unteren Drehzahlbereich von Motor 38 und Rotor 10 erfolgt der Betrieb "mit Fanglager", d.h. das Fanglager 56 befindet sich in der oberen Stellung. Gleichzeitig wird das Pendelgehäuse 40 durch die radial anfahrenden Spindeln 66 starr mit dem Maschinengestell 34 verbunden; damit ist dem Motorpendel seine Beweglichkeit genommen. Ferner werden die Lage des Kardangelenkes 42 und die Endpositionen der vier Spindeln 64 so justiert, daß die Bewegung des Rotorschwerpunktes bei der Umschaltung des Fanglagers in eine der beiden Stellungen stets kleiner als 0.02 mm bleibt. Zur Kontrolle können Wegmeßsonden mit einer Reproduzierbarkeit von ca. 2 $\mu$m, und zwar je zwei am Rotor 10 und am Pendelgehäuse 40, verwendet werden.

Die strichpunktierte Linie in Fig.3 zeigt den Instabilitätsbereich 68 des flüssigkeitsgefüllten Rotors "ohne Fanglager", der im niedrigen Drehzahlbereich auftreten würde, während die gestrichelte Linie den Instabilitätsbereich 70 des flüssigkeitsgefüllten Rotors "mit Fanglager", der im oberen Drehzahlbereich auftreten würde, erkennen läßt. Dazwischen befindet sich ein schmaler Drehzahlbereich, in dem sich der Rotor sowohl "mit" als auch "ohne Fanglager" stabil dreht. In diesem Bereich wird die aus Fig.3 erkennbare Umschaltdrehzahl gewählt, bei der beim Hochfahren vom Betrieb "mit Fanglager", d.h. das Fanglager 56 befindet sich in der oberen Stellung gemäß Fig.2, umgeschaltet wird auf den Betrieb "ohne Fanglager". Dadurch erhält man als Resultierende die Linie 72 in Fig.3, welche einen stabilen Betrieb des Rotors bei allen vorkommenden Drehzahlen angibt.

Der biegeweiche Abtriebswellenstumpf 50 ermöglicht es, die eine biegekritische Drehzahl des Rotorsystems und den daran gebundenen Instabilitätsbereich niedrig zu halten, so daß auch die Lagerkräfte im unteren Drehzahlbereich klein gehalten werden können. Bei den überkritischen Drehzahlen im oberen Drehzahlbereich tritt Selbstzentrierung, auch beim flüssigkeitsgefüllten Rotor, ein und das begrenzt die Lagerkräfte auf niedrige Werte.

Der Ölring 28 in der unteren Rinne 20 dient der Dämpfung des Systems in Bezug auf diejenigen Eigenschwingungen, die durch umlaufende Wellen im Flüssigkeitsring 26 entstehen. Die eigentliche Instabilität des flüssigkeitsgefüllten Rotors, die "ohne Fanglager" im unteren Drehzahlbereich liegt, kann aber durch die Ölringdämpfung nicht verhindert werden, sondern wird durch die oben erläuterte Fanglager-Umschaltung beseitigt.

Eine weitere Dämpfung wird im Zusammenhang mit der oben erwähnten, an sich bekannten pendelnden Abstützung des Motors 38 in dem Maschinengestell 34 und einer genau eingestellten Federung 46 bzw. Ölspaltdämpfungskammer 44 bewirkt (Fig.2). Diese Dämpfung hat den Zweck, beim Ausschalten des Fanglagers 56, also im oberen Drehzahlbereich, diejenigen Eigenschwingungen des Rotorsystems zu unterdrücken, die auch ohne Flüssigkeitsfüllung auftreten würden, insbesondere Biegeschwingungen und Präzession.

Eine einfach herstellbare Wellenkonstruktion besteht darin, den biegeweichen Abtriebswellenstumpf 50 in das obere Ende der biegesteifen Motorwelle 48 übergehen zu lassen.

Vorteilhaft wird jedoch gemäß Fig.2 eine aufwendigere Wellenkonstruktion vorgesehen. Dabei wird der immer noch biegeweiche Abtriebswellenstumpf 50 möglichst lang (300 mm) und dick (24 mm Durchmesser) ausgeführt und mit der jetzt hohlen Motorwelle 48 erst am unteren Ende durch Schrumpfsitz steif verbunden. Die angegebenen Maße ergeben sich aus der zur Verfügung stehenden Bauhöhe und der gewählten biegekritischen Drehzahl des Systems. Die beiden Wellen unterscheiden sich nach dem Herstellungsaufwand und der maximal erreichbaren Dämpfung der Präzession des Systems wie folgt: Die einfache Welle erlaubt bei etwa 10-facher biegekritischer Drehzahl von 9000 U/min nur eine Präzessionsdämpfung, die einer meßbaren Amplitudenabnahme von 100 auf 90% in 1 Sekunde entspricht; die Welle nach Fig.2 läßt jedoch eine Dämpfung entsprechend einer Amplitudenabnahme von 100 auf 30% in 1 Sekunde zu und erlaubt damit einen gefahrlosen Betrieb des Rotors 10 bis zu dessen höchstzulässiger Drehzahl. Die zuletzt dargestellten Verhältnisse leiten sich von den im Prinzip bekannten Eigenschaften eines Kreisels auf elastischer Welle ab und wurden versuchmäßig an dem hier beschriebenen Rotorsystem mit Fanglagerumschaltung entwickelt.

Wegen der möglichen Schwerpunktdifferenzen zwischen festem Rotor 10, Flüssigkeitsring 26 bzw. Ölring 28, die auf Herstellungstoleranzen der Rotorform an der Rinne 16 bzw. 20 beruhen, rufen die flüssigen Massen Unwuchten hervor, die sich durch Auswuchten am Rotor 10 nicht kompensieren lassen, weil sie asynchron sowohl gleich- als auch gegensinnig umlaufen können. Unter diesen Umständen können kleine Lager- bzw. Querkräfte zwischen Abtriebswellenstumpf 50 und Paßbohrung 54 nur bei überkritischer hoher Drehzahl oder bei unterkritischer niedriger Drehzahl eingehalten werden. Das gerade wird durch die Fanglagerumschaltung möglich, weil eine kritische Drehzahl nicht durchfahren wird, wie oben anhand Fig.3 erläutert wurde.

Der schon erwähnte Kessel 19, 21, 25 (Fig.2, 5) zur Herstellung eines Grobvakuums ist mit dem Rotorsystem schwingungsmäßig gekoppelt und muß in Bezug auf seine Masse und im Zusammenhang mit der Biegesteifigkeit des Maschinengestells 34 so dimensioniert werden, daß die resultierende Resonanzfrequenz mindestens 30% über der Fanglagerumschaltdrehzahl liegt; diese beträgt im Ausführungsbeispiel 2100 U/min. Andernfalls würde zusätzlich ein instabiler Drehzahlbereich des flüssigkeitsgefüllten Rotors bei Fanglager 56 "oben" und in der Umgebung der Fanglagerumschaltdrehzahl entstehen. Ein Durchfahren dieser Instabilität wäre nicht möglich.

Bei der in Fig.5 gezeigten Vorrichtung wird die Drehung des Gases über dem Rotor 10 vermindert und dadurch kann auch ein mit wenigen cm/s herabfallender Tropfennebel 30 verlustfrei in den rotierenden Flüssigkeitsring 26 gebracht werden. Eine lange Fallzeit des langsam herabschwebenden Tropfennebels 30 begünstigt, besonders bei langsam spreitender Suspension, das vollständige Spreiten an der Tropfenoberfläche und das Entstehen eines hohen Filmdrucks.

Über dem Rieselfilm 24 auf dem rotierenden Boden 12 wird zwangsweise eine wenige mm dicke Gas-Grenzschicht zum Mitrotieren und radialen Abfließen gebracht, so daß an der Innenseite des Flüssigkeitsrings 26 eine stark drehende Gasströmung hochgepumpt wird. Nach dem Aufsteigen an der Kesselinnenwand, wo sie durch Reibung etwas an Drehgeschwindigkeit verliert, verteilt sie sich, wenn keine weiteren Einbauten vorgesehen sind, in den Kesselinnenraum und fließt langsam und mit mäßiger Drehung zum Rotorboden 12 zurück. Die so entstehende Gasdrehströmung reicht erfahrungsgemäß aus, den feinen Tropfennebel 30 mit Tropfen-Durchmessern zwischen 60 und 20 $\mu$m an die Kesselwand zu schleudern und so der Mischung im Flüssigkeitsring 26 zu entziehen.

Im Ausführungsbeispiel sind ein Schaufelkranz 142 und ein Leitzylinder 143, 144 vorgesehen, die der Kesselinnenwand 25 unmittelbar anliegen. Der Schaufelkranz 142 lenkt die aufsteigende starke Drehströmung des Gases zuerst in die Vertikale nach oben um, wobei ein Druckgewinn entsteht, der bei der Rückführung des Gasstroms, größtenteils nach unten, durch Richtungsänderung und Reibung aufgebraucht wird (Fig.5).

Zwischen dem unteren Teil des Leitzylinders 143 und dem Schaufelkranz 142 sind vertikale Bleche angeordnet, zwischen denen der Gasstrom drehungsfrei nach unten abfließt; unten ist eine Vermischung mit dem weiter außen aufsteigenden Gasstrom wegen der Fliehkraftschichtung nicht möglich. Deshalb wird der unten ankommende Gasstrom über dem rotierenden Boden 12 zuerst von außen nach innen verteilt, dann wieder in die mitrotierende Grenzschicht gesaugt und nach außen abgeschleudert. Auf diese Weise kann das Gasvolumen zwischen Drehachse 14 und Leitzylinder 143, 144 bis auf die dünne Gas-Grenzschicht drehungsfrei gehalten werden.

Ein kleinerer Anteil des den Schaufelkranz 142 verlassenden Gasstromes fließt zwischen dem oberen Teil des Leitzylinders 144 bzw. Umlenkblech 146 und Glaskessel 211 auf dem Weg 147 aus und wird in der darüber angeordneten Sprüheinrichtung benötigt.

Im folgenden wird eine zweckmäßige Ausführungsform der Sprüheinrichtung nach Fig.5 und 7 beschrieben. Sie wird in einen Glaskessel 121 mit Deckelflansch 122 eingebaut, der im unteren, engeren Teil etwa den gleichen Innendurchmesser wie der Flüssigkeitsring 26 im Rotor 10 aufweist. Der oben erwähnte kleinere Gasstrom 147 geht dem von oben auf dem Weg 156 herabkommenden Tropfennebel entgegen und verhindert eine Wandberührung an der Verengung des Glaskessels 121.

Die Sprüheinrichtung enthält in der Mitte eine Sprühscheibe 80, die mit 10000 bis 27000 U/min rotiert. Ihrer Unterseite 84 wird die zweite Suspension über eine Doppeldüse 92 zugeführt, so daß am Scheibenrand feine Tröfchen, vorzugsweise mit 60 bis 20 $\mu$m Durchmesser je nach Scheibendrehzahl, entstehen.

In einer besonders vorteilhaften Ausführung gemäß Fig.5 werden Oberseite 88 und Unterseite 84 als doppelt gewellte Fläche gestaltet, so daß die am Rand mit einigen 100 m/s abfliegenden Tröpfchen sich auf die freie Höhe eines in Fig.5 gezeigten Treibkanals 154 bzw. Sprühkanals 152 verteilen.

Die aus einem Treibwasserzufluß 86 an der Oberseite 88 gebildeten Tröpfchen dienen dazu, einen Gasstrom durch den Treibkanal 154 zu treiben. Das Gas wird durch Siebbleche 133 hindurchgedrückt und strömt zum größten Teil durch den Sprühkanal 152 zurück zum Treibkanal 154. Dadurch wird, zusammen mit der sehr gleichmäßigen Dosierung der Zuflüsse 82, 86 erreicht, daß die aus der zweiten Suspension gebildeten Tröpfchen von ihrer hohen Geschwindigkeit am Sprühscheibenrand abgebremst werden, um dann in den nächsten Kreislauf der Gasströmung 156, 158 zu gelangen, der eine etwa 10- bis 50-fach kleinere Förderleistung (in l/s) aufweist als der erstgenannte durch Treib- und Sprühkanal. Der kleine, vom Treibkanal 154 auf den Weg 156 übergehende Gasstrom zweigt weiter unten ab über den Weg 149, das regulierbare Axialgebläse 137 und die Wege 150, 151. Auf diese Weise sind einerseits der erste und zweite Kreislauf der Gasströmung verflochten, d.h. die Tröfchen werden vom ersten zum zweiten Kreislauf transportiert, andererseits können die Tröpfchen für eine gewisse Zeit innerhalb des zweiten Kreislaufs verweilen, um das langsame Spreiten der zweiten Suspension an die Tröpfchenoberfläche zu ermöglichen. Dieses könnte durch ein direktes Einspritzen des Tropfennebels 30, z.B. durch Sprühdüsen, in den Flüssigkeitsring 26 keinesfalls erreicht werden.

Aus dem zweiten, langsamen Kreislauf 156, 158 fallen die Tröpfchen längs der Pfeile 148 bis auf die oben erwähnte Gasgrenzschicht über dem Rotorboden 12, von der sie in den Flüssigkeitsring getragen werden, ohne den Rieselfilm 24 zu berühren. Um zu verhindern, daß die Tröpfchen in den Gasstrom durch das Axialgebläse 137 mitgerissen werden, wird das untere Ende des Trichterblechs 136 mit einem Blechzylinder, in Fig.5 nicht gezeichnet, bis auf wenige cm Abstand zum Rotorboden 12 verlängert.

Gemäß Fig.5, 7 wird die zweite Suspension der Sprühscheiben-Unterseite 84 in folgender Art zugeführt: Durch den inneren Teil 81 einer Doppeldüse 82 wird ein zentraler Strahl aus Pufferlösung und eine Grundschicht auf der Scheibe gebildet, während die zu versprühende Suspension über den äußeren Teil 83 der Doppeldüse als Deckschicht zufließt und bis zum Scheibenrand sehr dünn ausgezogen wird. Die Suspension gelangt so nach dem Absprühen vom Scheibenrand konzentriert an die Tröpfchenoberfläche, wo sie schneller spreiten kann als bei einheitlichem Zufluß. Diese Möglichkeit bietet nur das Versprühen über eine rotierende Scheibe, nicht dagegen das durch Ultraschallvernebler oder Druck-beaufschlagte Sprühdüsen. Dafür muß allerdings das komplizierte Abbremsen der abgeschleuderten Tröpfchen im dargestellten Sprühkanal 152 in Kauf genommen werden; außerdem muß eine genaue Einstellung und Dosierung der beiden Zuflüsse zur Scheiben-Unter-und Oberseite 84, 88 gesichert werden.

Vorteilhaft wird gemäß Fig.5 eine Vermischung der den Sprühkanal 152 verlassenden Tröpfchen mit denen aus dem Treibkanal 154 vermieden, indem letztere am Außenrand durch kreisbogenförmige Schaufeln 140, die mit ca. 20 mm Abstand über den Umfang verteilt stehen und in rechtem Winkel an der Innenwand des Glaskessels 121 aufsitzen, in streifendem Aufprall abgefangen und abgeleitet werden.

**Patentansprüche**

1. Verfahren zur Herstellung wäßriger Kolloide durch Mischen zweier Suspensionen in etwa gleichen Anteilen, die jeweils amphiphile und lipophile Bestandteile sowie eine wäßrige Pufferlösung enthalten, dadurch gekennzeichnet, daß aus der ersten Suspension ein dünner Rieselfilm und aus der zweiten Suspension ein feiner Tropfennebel gebildet wird, daß der Rieselfilm bezüglich des Tropfennebels in annähernd horizontaler Ebene in schnelle Drehung versetzt und an seinem Umfang in Form eines rotierenden Flüssigkeitsringes aufgefangen wird und daß der Tropfennebel mit der Oberfläche des Rieselfilms auf dem rotierenden Flüssigkeitsring in Berührung und zur Mischung gebracht wird.

2. Verfahren nach Anspruch 1, dadurch gekennzeichnet, daß die je Zeiteinheit gebildeten Oberflächen von Rieselfilm und Tropfennebel ungefähr gleich groß gemacht, und die zugeführten Suspensionen auf diesen Oberflächen mit einem Filmdruck von 5 bis 25 dyn/cm ($5 \cdot 10^{-5}$ - $2,5 \cdot 10^{-4}$ N/cm) zum Spreiten gebracht werden.

3. Verfahren nach Anspruch 1 und 2, dadurch gekennzeichnet, daß die Drehbewegung des rotierenden Flüssigkeitsringes, ausgehend von einer Anfangsdrehzahl, während der Berührung mit dem Tropfennebel ständig leicht verzögert wird, bis die Drehzahl um 10% bis 50% verringert ist, und daß gleichzeitig dem Rieselfilm in gleichem Verhältnis mehr Pufferlösung zugeführt wird.

4. Verfahren nach einem der vorangehenden Ansprüche, dadurch gekennzeichnet, daß die erste und/oder zweite Suspension bei der Bildung des Rieselfilms bzw. Tropfennebels verdünnt und dazu mindestens zweifach konzentrierter, verglichen mit ihrer Konzentration in der zuletzt entstehenden Mischung, zugegeben werden, derart, daß Rieselfilm und Tropfennebel zu einem größeren Volumenanteil aus Pufferlösung gebildet werden, während die zugehörigen Suspensionen mit dem restlichen Volumenanteil als Deckschicht an der Oberfläche des Rieselfilms bzw. der Tropfen aufgebracht werden.

5. Vorrichtung zur Durchführung des Verfahrens nach einem der vorangehenden Ansprüche, mit je einer Dosiereinrichtung für die Zuführung der beiden Suspensionen, gekennzeichnet durch einen im Querschnitt etwa scheibenförmigen, oben eine offene Schale (11) bildenden Rotor (10) mit flachem Boden (12) zur Aufnahme des Rieselfilms (24) und um den Umfang des Bodens (12) laufender, zur Rotorachse (14) hin offener, in den Boden (12) übergehender erster ringförmiger Rinne (16) zur Aufnahme des rotierenden Flüssigkeitsrings (26), wobei der Rotor (10) auf dem vertikalen Abtriebswellenstumpf (50) eines Motors (38) fliegend gelagert ist, durch eine mit der Dosiereinrichtung für den Rieselfilm (24) in Verbindung stehende Zuleitungsdüse (22) in der Drehachse (14) des Rotors (10) über dessen Boden (12), durch ein mit der Dosiereinrichtung für den Tropfennebel (30) in Verbindung stehende Sprüheinrichtung oberhalb des Rotorbodens (12) und durch Stabilisierungseinrichtungen für den Rotor (10).

6. Vorrichtung nach Anspruch 5, dadurch gekennzeichnet, daß der Abtriebswellenstumpf (50) aus einem Stahl hoher Festigkeit gegen Biegung und Torsion besteht, bei einem Durchmesser von 24 mm eine freie Länge von 300 mm aufweist und mittels Schrumpfsitz am unteren Ende mit der hohlen Antriebswelle (48) biege- und torsionssteif verbunden ist.

7. Vorrichtung nach Anspruch 5 oder 6, dadurch gekennzeichnet, daß am oberen Ende (52) des Abtriebswellenstumpfes (50) ein parallel zur Rotordrehachse (14) verschiebbares und durch Verschieben umschaltbares Fanglager (56) vorgesehen ist, das bei niedriger Drehzahl des Rotors (10) an einen mit dem Abtriebswellenstumpf (50) fest verbundenen Konus (64) anlegbar ist, während es nach Umschaltung bei hohen Drehzahlen den Konus (64) freigibt.

8. Vorrichtung nach einem der Ansprüche 5 bis 7, dadurch gekennzeichnet, daß an der Unterseite (18) des Rotors (10) im Umfangsbereich der den Flüssigkeitsring (26) aufnehmenden ersten Rinne (16) eine zweite ringförmige Rinne (20) vorgesehen ist, die mit einer Dämpfungsflüssigkeit (28) hoher Zähigkeit und Dichte gefüllt ist.

9. Vorrichtung nach einem der Ansprüche 5 bis 8, dadurch gekennzeichnet, daß der Motor (38) innerhalb eines Motorgehäuses (40) in einem Maschinengestell (34) pendelnd, federnd (46) und ölgedämpft (44) derart gelagert ist, daß eine z.B. durch Stoß angeregte Präzession des Rotors (10) bei Maximaldrehzahl, die etwa das zehnfache der biegekritischen Drehzahl beträgt, eine Amplitudenabnahme je Sekunde auf weniger als 30% aufweist.

10. Vorrichtung nach Anspruch 9, dadurch gekennzeichnet, daß das Motorgehäuse (40) durch vier Spindeln (66) zugleich mit dem Hochschieben des Fanglagers (56) festlegbar ist.

11. Vorrichtung nach Anspruch 9 oder 10, dadurch gekennzeichnet, daß die Biegesteifigkeit des auf einem Fundament (36) verschraubten Maschinengestells (34) bezüglich horizontaler Auslenkung bzw. die entsprechende erste Resonanzfrequenz des Maschinengestells (34) so hoch bemessen sind, daß letztere wenigstens 30% über der Umschaltdrehzahl des Fanglagers (56) liegt.

12. Vorrichtung nach einem der Ansprüche 5 bis 11, dadurch gekennzeichnet, daß die Zuleitungsdüse (22) das untere Ende einer zylindrischen, unten mit einer konischen Verjüngung (77) versehenen und innen hohlen Filterkerze (74) bildet, das in eine ringförmige Schneide (73) übergeht und in der Drehachse (14) im Abstand von wenigen mm über dem rotierenden Boden (12) hängt.

13. Vorrichtung nach einem der Ansprüche 5 bis 12, dadurch gekennzeichnet, daß der aus einer Aluminiumlegierung gefertigte Rotor (10) bei einem wählbaren Außendurchmesser D folgende Maße besitzt:
eine größte Dicke H, gemessen über den Flüssigkeitsring (26) bzw. Ölring (28), zwischen H = $0{,}136 \cdot D$ und $0{,}150 \cdot D$,
eine größte Dicke G in Scheibenmitte zwischen G = $0{,}114 \cdot D$ und $0{,}139 \cdot D$
eine kleinste Dicke h am Scheibenrand zwischen h = $0{,}0306 \cdot D$ und $0{,}0333 \cdot D$,
eine kleinste radiale Dicke a an der Rinne (16) zwischen a = $0{,}0278 \cdot D$ und $0{,}0306 \cdot D$ bzw. b an der Rinne (20) zwischen b = $0{,}0278 \cdot D$ und $0{,}0333 \cdot D$,
eine radiale Tiefe c der Rinne (16) zwischen c = $0{,}0278 \cdot D$ und $0{,}0417 \cdot D$ bzw. d der Rinne (20) zwischen d = $0{,}0306 \cdot D$ und $0{,}0444 \cdot D$,
eine freie Höhe k der Rinne (16) zwischen k = $0{,}0486 \cdot D$ und $0{,}0611 \cdot D$ bzw. 1 der Rinne (20) zwischen l = $0{,}0333 \cdot D$ und $0{,}0361 \cdot D$,
einen Krümmungsradius e am Übergang des Rotorbodens (12) zur Rinne (16) zwischen e = $0{,}0250 \cdot D$ und $0{,}0278 \cdot D$ bzw. f am Übergang der Rotorunterseite (18) zur Rinne (20) zwischen f = $0{,}0194 \cdot D$ und $0{,}0208 \cdot D$,
am Rotorboden (12) ein Gefälle $\alpha$ zwischen etwa 6,0 und 8,5° radial nach außen, das erst kurz vor dem Flüssigkeitsring (26) endet.

14. Vorrichtung nach einem der Ansprüche 5 bis 13, dadurch gekennzeichnet, daß ein Schaufelkranz (142) mit z.B. kreisförmig gebogenen Schaufelblechen zum Auffangen und Umlenken annähernd in die Vertikale einer an der Innenseite des rotierenden Flüssigkeitsringes (26) in flacher Spirale aufsteigenden Gasströmung sowie ein Leitzylinder (143, 144) zum Zurückleiten des größten Teiles der Gasströmung in das Volumen über dem Rotorboden (12) und des Restes entlang der Innenwand (147) eines Glaskessels (121) nach oben vorgesehen sind.

15. Vorrichtung nach einem der Ansprüche 5 bis 14, dadurch gekennzeichnet, daß die zweite Suspension über einen Zufluß (82) an der Unterseite (84) einer in der Achse (14) unter einem Deckelflansch (122) hängend gelagerten und mit konstanter Drehzahl rotierenden Sprühscheibe (80), und zugleich eine wässrige Pufferlösung über einen Zufluß (86) an der Oberseite (88) der Sprühscheibe (80), jeweils zur Bildung eines über die Schiebenfläche zusammenhängenden Flüssigkeitsfilms, zuführbar sind.

16. Vorrichtung nach Anspruch 15, dadurch gekennzeichnet, daß zum Abbremsen der durch die Sprühscheibe (80) versprühten zweiten Suspension ein ringförmiger Sprühkanal (152) vorgesehen ist, oben begrenzt von einer Zwischenplatte (132) bzw. von Siebblechen (133), unten begrenzt von einer Bodenplatte (134), und zum Abbremsen der durch die Scheibe (80) versprühten wässrigen Pufferlösung ein ringförmiger Treibkanal (154) vorgesehen ist, oben begrenzt von einer Deckplatte (130) und unten von der Zwischenplatte (132) bzw. den Siebblechen (133).

17. Vorrichtung nach Anspruch 16, dadurch gekennzeichnet, daß zum Abziehen eines kleinen Teils (149) des aus dem Treibkanal (154) zum Sprühkanal (152) zurückströmenden Gases und zum Ausblasen desselben über ein Trichterblech (136), oben begrenzt durch die Bodenplatte (134), auf Wegen (150, 151) im oberen Teil des Glaskessels (121) ein in der Drehachse (14) in mittlerer Höhe des Glaskessels (121) angebrachtes Axialgebläse (137) vorgesehen ist.

18. Vorrichtung nach einem der Ansprüche 15 bis 17, dadurch gekennzeichnet, daß Unterseite (84) und Oberseite (88) der Sprühscheibe (80) je mit zwei diametral liegenden Rippen (87) und zwei um 90° dazu versetzten Senken (89) versehen sind, wobei die verbindende Fläche als ringförmige Doppelwelle erscheint und am Außenradius den größten Höhenunterschied, entsprechend mindestens der halben Höhe von Sprühkanal (152) bzw. Treibkanal (154), dagegen etwa beim halben Radius keinen solchen Höhenunterschied mehr aufweist.

19. Vorrichtung nach einem der Ansprüche 15 bis 18, dadurch gekennzeichnet, daß der Zufluß (82) in wenigen mm Abstand vom Zentrum der Sprühscheibenunterseite (84) und um etwa 30° aus der Vertikalen verdreht angebracht ist und aus einer zweifachen, konzentrischen Düse besteht, deren innerer Teil (81) mit einer Zuleitung für wässrige Pufferlösung und deren Mantelteil (83) mit einer Zuleitung für die zweite Suspension verbunden ist.

20. Vorrichtung nach einem der Ansprüche 16 bis 10, dadurch gekennzeichnet, daß zum Abfangen der von der Oberseite (88) der Sprühscheibe (80) in den Treibspalt (154) versprühten Tröpfchen in streifendem Aufprall und zum Ableiten derselben auf die Inneseite des Glaskessels (121) vertikal stehende, kreisförmig gebogene Schaufeln (140) am Außenradius des Treibspalts (154) vorgesehen sind.

## Claims

1. A method for producing aqueous colloids by mixing in equal parts two suspensions each containing amphiphilic and lipophilic constituents as well as an aqueous buffer solution, characterized in that a thin trickling film is formed from the first suspension and a fine mist of droplets from the second suspension, in that said trickling film is set into quick rotary motion in an approximately horizontal plane with respect to said mist of droplets and is collected at its periphery in the form of a rotating liquid ring, and in that said mist of droplets is brought into contact and mingled with the surface of said trickling film on said liquid ring.

2. A method according to claim 1, characterized in that the surface areas generated per unit of time by said trickling film and by said mist of droplets are rendered approximately equal, and in that the suspensions applied onto said surfaces are made to spread thereon with a film pressure of 5 to 25 dynes/cm (5 · $10^{-5}$ to 2.5 · $10^{-4}$ N/cm).

3. A method according to claims 1 and 3, characterized in that the rotaty motion of said rotating liquid ring, starting from an initial speed, is progressively slowed down by small amounts during said contacting operation with said mist of droplets until its speed will have decreased by 10% to 50%, and in that at the same time the supply of buffer solution to said trickling film is increased at an inversely proportional rate.

4. A method according to any of the preceding claims, characterized in that said first and/or the second suspension for the formation of said trickling film and of said mist of droplets respectively are added in a dilution and at least twice more concentrated as compared with their final concentration in the last produced mixture, in such a way as to form said trickling film and said mist of droplets with a major volume fraction of buffer solution, whereas the related suspensions are applied to the surfaces of said trickling film and of said mist of droplets respectively with the remaining volume fraction.

5. A device for performing the method according to any of the preceding claims, comprising a dosage unit for adding each of said two suspensions, characterized by a rotor (10) with an approximately disk-shaped cross section forming a cup (11) open on its top, with a flat bottom (12) for collecting said trickling film (24) and with a first ring-shaped groove (16) surrounding the periphery of said bottom (12), opening towards the axis (14) of said rotor and verging into said bottom (12) for receiving said rotating liquid ring (26), said rotor (10) being overhung-mounted on the driven shaft end (50) of a motor (38), by a feed nozzle (22) communicating with said dosage unit for said trickling film (24) in said rotational axis (14) of rotor (10) through its bottom (12), by a spray unit above said bottom (12) of said rotor communicating with said dosage unit for said mist of droplets (30), and by stabilizing systems for said rotor (10).

6. A device according to claim 5, characterized in that said driven shaft end (50) is made of a high flectional and torsional strength steel with a diameter of 24 mm and a free length of 300 mm and is connected at its lower end to the hollow drive shaft (48) by shrink fit in a flection and torsion proof manner.

7. A device according to claim 5 or 6, characterized in that a restraining bearing (56) is provided at the head (52) of said driven shaft end allowing displacement thereof parallel to said rotational axis (14) of said rotor, and is reversed by said displacement, said restraining bearing being engageable with a cone (64) integral with said driven shaft end (50) at low speed of rotor (10), whereas it releases said cone (64) after reversal at high speed.

8. A device according to any of the claims 5 to 7, characterized in that a second ring-shaped groove (20) is provided on the bottom side (18) of rotor (10) in the peripheral area of said first groove (16) receiving said liquid ring (26), which is filled with a damping fluid (28) of high viscosity and density.

9. A device according to any of the claims 5 to 8, characterized in that said motor (38) runs on self-aligning elastic (46) and oil-damped (44) bearings inside a motor casing (40) in a machine frame (34) in such a way that any precession of rotor (10) e.g. generated by shocks at maximum speed, which is approximately ten times the critical whirling speed, undergoes a reduction of amplitude per second to less than 30%.

10. A device according to claim 9, characterized in that said motor housing (40) can be locked by four screw actuators (66) simultaneously with pushing upwards said restraining bearing (56).

11. A device according to claim 9 or 10, characterized in that the flectional strength of said machine frame (34) screwed on a foundation (36) in regard of horizontal deviation, or the corresponding resonance frequency of said machine frame (34) respectively, are made as high as to place the latter at least 30% above the reversing speed of said restraining bearing (56).

12. A device according to any of the claims 5 to 11, characterized in that said feed nozzle (22) forms the low end of a zylindrical and hollow filter plug (74) provided with a taper (77) at its lower end, turning into an annular cutting edge (73) and being suspended in the axis (14) of rotation at a distance of a few millimeters above the rotating bottom (12).

13. A device according to any of the claims 5 to 12, characterized in that said rotor (10) made of an aluminium alloy has the following dimensions, with a random outside diameter D:
a maximum thickness H, measured across the liquid ring (26) or the oil ring (28) respectively, comprised between $H = 0.136 \cdot D$ and $H = 0.150 \cdot D$,
a maximum thickness G in the center of the disk, comprised between $G = 0.114 \cdot D$ and $G = 0.139 \cdot D$,
a minimum thickness h at the rim of the disk comprised between $h = 0.0306 \cdot D$ and $h = 0.0333 \cdot D$,
a minimum radial thickness a at groove (16) comprised between $a = 0.0278 \cdot D$ and $a = 0.0306 \cdot D$, and b at groove (20) respectively, comprised between $b = 0.0278 \cdot D$ and $b = 0.0333 \cdot D$,
a radial depth c of groove (16) comprised between $c = 0.0278 \cdot D$ and $c = 0.0417 \cdot D$, and d of groove (20) respectively, comprised between $d = 0.0306 \cdot D$ and $d = 0.0444 \cdot D$,
a clearance hight k of groove (16) comprised between $k = 0.0486 \cdot D$ and $k = 0.0611 \cdot D$, and 1 of groove (20) respectively, comprised between $1 = 0.0333 \cdot D$ and $1 = 0.0361 \cdot D$,
a radius of curvature e at the transition from the bottom (12) of the rotor to groove (16) comprised between $e = 0.0250 \cdot D$ and $e = 0.0278 \cdot D$, and f at the transition from the lower side (18) of the rotor to groove (20) respectively, comprised between $f = 0.0194 \cdot D$ and $f = 0.0208 \cdot D$, and
at the bottom (12) of the rotor, a slope $\alpha$ comprised between approx. 6.0° and 8.5° radially outwards, terminating short of liquid ring (26).

14. A device according to any of the claims 5 to 13, characterized by a blade ring (142) having its blades e.g. curved in the form of an arc of a circle, for collecting a gas flow rising at the inner face of said rotating liquid ring (26) in a flat spiral line and for deflecting it into approximately vertical direction, and by a guide cylinder (143, 144) for returning the bulk of said gas flow into the volume above bottom (12) of the rotor, and the remainder upwards along the inner wall (147) of a glass vessel (121).

15. A device according to any of the claims 5 to 14, characterized in that said second suspension can be supplied through an inlet (82) at the bottom side (84) of a spraying disk (80) suspended in the axis (14) below a blank flange (122) and rotating at constant speed, an aqueous buffer solution being at the same time supplied through an inlet (86) at the upper side (88) of said spraying disk (80), and both being intended to form a coherent liquid film throughout the surface of said disk.

16. A device according to claim 15, characterized by an annular spray channel (152) provided for decelerating said second suspension sprayed by said spraying disk (80), delimited on its upper side by an intermediate plate (132) and by mesh plates (133) respectively and on its lower side by a bottom plate (134), and by an annular driving channel (154) provided for decelerating the aqueous buffer solution sprayed by said disk (80), delimited on its upper side by a cover plate (130) and on its lower side by said intermediate plate (132) and by said mesh plates (133) respectively.

17. A device according to claim 16, characterized by an axial blower (137) provided in the axis (14) of rotation at medium hight of said glass vessel (121) for extracting a small portion (149) of the gas returning from said driving channel (154) to said spray channel (152) and for evacuating it, through a funnel plate (136) delimited on its upper side by said bottom plate (134), on paths (150, 151) in the upper part of said glass vessel (121).

18. A device according to any of the claims 15 to 17, characterized in that the bottom side (84) and the upper side (88) of said spraying disk (80) are provided with two diametrical ribs (87) and with two sinks (89) offset by 90° with reference to the latter, the joining surface appearing as a ring-shaped double wawe having its greatest level difference on its periphery, corresponding at least to half the height of spraying channel (152) or of driving channel (154) respectively, such a level difference no longer existing at about half the radius.

19. A device according to any of the claims 15 to 18, characterized in that said inlet (82) is placed at a disstance of a few millimeters from the center of the bottom side (84) of said spraying disk, inclined against the vertical position by approximately 30°, and consists of a concentric double nozzle, the inner part (81) of which communicates with a supply line for the aqueous buffer solution, its enveloping part (83) communicating with a supply line for the second suspension.

20. A device according to any of the claims 16 to 19, characterized in that blades (140) curved in the form of arcs of a circle are provided in the peripheral area of driving channel (154) to collect the droplets sprayed from the upper side (88) of spraying disk (80) into said driving channel (154), by grazing impact, and to deviate them onto the inner face of said glass vessel (121).

**Revendications**

1. Procédé pour la fabrication de colloïdes aqueux par mélange de deux suspensions à parts sensiblement égales, contenant des composants amphiphiles et lipophiles ainsi qu'une solution tampon aqueuse, caractérisé en ce qu'on produit un film ruisselant mince à partir de la première suspension et un embrun de gouttelettes à partir de la deuxième suspension, que le film ruisselant est mis en mouvement de rotation rapide dans un plan approximativement horizontal par rapport à l'embrun de gouttelettes et capté à sa périphérie sous forme d'un anneau liquide tournant, et que l'embrun de gouttelettes est mis en contact et mélangé avec la surface du film ruisselant sur l'anneau liquide tournant.

2. Procédé selon la revendication 1, caractérisé en ce que les surfaces du film ruisselant et de l'embrun de gouttelettes formées par unité de temps sont rendues sensiblement égales, et que les suspensions ajoutées sont amenées à se répandre sur ces surfaces avec une pression du film d'env. 5 à 25 dynes/cm ($5 \cdot 10^{-5}$ à $2,5 \cdot 10^{-4}$ N/cm).

3. Procédé selon les revendications 1 et 2, caractérisé en ce que le mouvement de rotation de l'anneau liquide tournant est légèrement et progressivement décéléré à partir d'une vitesse initiale au cours du contact avec l'embrun de gouttelettes, jusqu'à ce que la vitesse soit réduite de 10% à 50%, et en ce que l'apport de la solution tampon au film ruisselant est en même temps augmenté dans la même proportion.

4. Procédé selon l'une quelconque des revendications précédentes, caractérisé en ce que la première et/ou la deuxième suspension sont ajoutées, lors de la formation du film ruisselant ou respectivement de l'embrun de gouttelettes, en dilution et au moins deux fois plus concentrées par rapport à leur concentration dans le mélange formé finalement, de manière à ce que le film ruisselant et l'embrun de gouttelettes soient formés avec une teneur volumétrique plus importante de la solution tampon, tandis que les suspensions associées sont appliquées à la surface du film ruisselant ou respectivement des gouttelettes comme couche superficielle avec la teneur volumétrique résiduelle.

5. Dispositif pour la réalisation du procédé selon l'une quelconque des revendications précédentes, comportant, pour l'amenée de chacune des deux suspensions, un mécanisme de dosage, caractérisé par un rotor (10) sensiblement en forme de disque dans sa section transversale, formant une cuvette (11) ouverte en haut, à fond plat (12) pour recevoir le film ruisselant (24), avec un premier caniveau annulaire (16) s'étendant le long de la circonférence du fond (12), s'ouvrant vers l'axe (14) du rotor et rejoignant le fond (12), pour contenir l'anneau liquide tournant (26), le rotor (10) étant monté en porte à faux sur le bout d'arbre d'entraînement (50) vertical d'un moteur (38), par une buse d'alimentation (22) en communication avec le mécanisme de dosage pour le film ruisselant (24) dans l'axe (14) du rotor (10) au-dessus du fond (12) de celui-ci, par un pulvérisateur en communication avec le mécanisme de dosage pour l'embrun de gouttelettes (30) au-dessus du fond (12) du rotor, et par des organes de stabilisation pour le rotor (10).

6. Dispositif selon la revendication 5, caractérisé en ce que le bout d'arbre d'entraînement (50) est fait d'un acier ayant und résistance élevée à la flexion et à la torsion, qu'il présente une longueur libre de 300 mm avec un diamètre de 24 mm, et qu'il est relié, à son bout inférieur, avec l'arbre menant creux (48) par ajustage à chaud de façon rigide à la flexion et à la torsion.

7. Dispositif selon l'une des revendications 5 et 6, caractérisé en ce qu un roulement amortisseur (56) réversible par coulissement parallèle à l'axe (14) du rotor est disposé à l'extrémité supérieure (52) du bout d'arbre d'entraînement (50) et peut s'appliquer contre un cône (64) solidaire du bout d'arbre d'entraînement (50) aux petites vitesses du rotor (10), tandis qu'il libère le cône (64) après renversement aux vitesses élevées.

8. Dispositif selon l'une quelconque des revendications 5, 6 et 7, caractérisé en ce qu'un deuxième caniveau annulaire (20) rempli d'un liquide amortisseur (28) à viscosité et densité élevées est disposé sur la face inférieure (18) du rotor (10) dans la zone périphérique du premier caniveau (16) recevant l'anneau liquide (26).

9. Dispositif selon l'une quelconque des revendications 5 à 8, caractérisé en ce que le moteur (38) contenu dans une carcasse de moteur (40) tourne sur paliers articulés élastiques (46) à amortissement par huile (44) dans un bâti de machine (34), de sorte qu'une précession du rotor (10) engendrée p.ex. par un choc à la vitesse maximum dont la valeur est env. dix fois plus élevée que la vitesse critique de rotation subisse une réduction d'amplitude par seconde à une valeur inférieure à 30%.

10. Dispositif selon la revendication 9, caractérisé en ce que la carcasse de moteur (40) est serrée par l'action de quatre tiges (66) en même temps que le roulement amortisseur (56) est poussé vers le haut.

11. Dispositif selon l'une des revendications 9 et 10, caractérisé en ce que la résistance à la flexion du bâti de machine (34) vissé sur un socle (36) par rapport à une déviation horizontale, ou respectivement la première fréquence de résonance correspondante du bâti de machine (34) soient suffisamment élevées pour que cette dernière soit placée au moins 30% au-dessus de la vitesse de renversement du roulement amortisseur (56).

12. Dispositif selon l'une quelconque des revendications 5 à 11, caractérisé en ce que la buse d'alimentation (22) forme le bout inférieur d'une bougie filtrante (74) cylindrique creuse pourvue d'une partie effilée en cône (77), qui se termine en arête vive annulaire (73) suspendue dans l'axe (14) de rotation à une distance de quelques millimètres au-dessus du fond (12) tournant.

13. Dispositif selon l'une quelconque des revendications 5 à 12, caractérisé en ce que le rotor (10) fabriqué en alliage d'aluminium présente, avec un diamètre extérieur D, les dimensions suivantes:
une épaisseur maximum H mesurée à travers l'anneau liquide (26) ou respectivement l'anneau d'huile (28), comprise entre H = 0,136·D et H = 0,150·D,

une épaisseur maximum G au centre du disque, comprise entre G = 0,114·D et G = 0,139·D,

une épaisseur minimum h au bord du disque, comprise entre h = 0,0306·D et h = 0,0333·D,

une épaisseur radiale minimum a du caniveau (16) comprise entre a = 0,0278·D et a = 0,0306·D, et respectivement b du caniveau (20) comprise entre b = 0,0278·D et b = 0,0333·D,

une profondeur radiale c du caniveau (16) comprise entre c = 0,0278·D et c = 0,0417·D, et respectivement d du caniveau (20) comprise entre d = 0,0306·D et d = 0,0444·D,

une hauteur libre k du caniveau (16) comprise entre k = 0,0486·D et k = 0,0611·D, et respectivement 1 du caniveau (20) comprise entre 1 = 0,0333·D et 1 = 0,0361·D,

un rayon de courbure e à la transition du fond (12) du rotor au caniveau (16) compris entre e = 0,0250·D et e = 0,0278·D, et respectivement f à la transition de la face inférieure (18) du rotor au caniveau (20) compris entre f = 0,0194·D et f = 0,0208·D et,

au fond (12) du rotor, une pente $\alpha$ comprise entre 6,0° et 8,5° radialement vers l'extérieur, qui ne se termine que peu avant l'anneau liquide (26).

14. Dispositif selon l'une quelconque des revendications 5 à 13, caractérisé par une couronne d'aubes (142) comportant des aubes en tôle p. ex. courbées en arc de cercle pour capter un courant de gaz ascendant en spirale plate sur la face intérieure de l'anneau liquide tournant (26) et pour le dévier sensiblement dans une direction verticale, et par un cylindre-guide (143, 144) pour ramener la plus grande partie du courant de gaz dans le volume au-dessus du fond (12) du rotor, et le reste vers le haut le long de la paroi intérieure (147) d'un vaisseau en verre (121).

15. Dispositif selon l'une quelconque des revendications 5 à 14, caractérisé en ce que la deuxième suspension est acheminée à travers une bouche (82) sur la face inférieure (84) d'un disque de pulverisation (80) suspendu dans l'axe (14) sous une bride d'obturation (122) et tournant à une vitesse constante, une solution tampon aqueuse étant en même temps dirigée à travers une bouche (86) sur la face supérieure (88) du disque de pulvérisation (80), ceci en vue de la formation d'un film de liquide cohérent sur la surface du disque.

16. Dispositif selon la revendication 15, caractérisé par un conduit de pulvérisation annulaire (152) prévu pour décélérer la deuxième suspension pulvérisée par le disque de pulvérisation (80), limité en haut par une plaque intermédiaire (132) ou respectivement par des tôles perforées (133) et limité en bas par une plaque de base (134), et par un conduit d'entraînement (154) annulaire prévu pour décélérer la solution tampon aqueuse pulvérisée par le disque de pulvérisation (80), limité en haut par une plaque de recouvrement (130) et en bas par la plaque intermédiaire (132) ou respectivement par les tôles perforées (133).

17. Dispositif selon la revendication 16, caractérisé par un ventilateur axial (137) disposé dans l'axe (14) à hauteur moyenne du vaisseau en verre (121) et prévu pour extraire une petite partie (149) du gaz refluant du conduit d'entraînement (154) vers le conduit de pulvérisation (152) et pour l'évacuer, à travers une tôle en forme d'entonnoir (136) limité en haut par la plaque de base (134), suivant des trajets (150, 151) dans la partie supérieure du vaisseau en verre (121).

18. Dispositif selon l'une quelconque des revendications 15 à 17, caractérisé en ce que la face inférieure (84) ainsi que la face supérieure (88) du disque de pulvérisation (80) sont chacune pourvues de deux nervures diamétrales (87) et de deux enfoncements (89) décalés de 90° par rapport à celles-ci, leur surface de jonction prenant la forme d'une onde double annulaire qui présente la différence de niveau maximum au rayon extérieur, correspondant au moins à la moitié de la hauteur du conduit de pulvérisation (152) ou respectivement du conduit d'entraînement (154), tandis qu'il n'y a plus une telle différence de hauteur à la moitié du rayon.

19. Dispositif selon l'une quelconque des revendications 15 à 18, caractérisé en ce que la bouche (82) est disposée à une distance de quelques millimètre seulement du centre de la face inférieure (84) du disque de pulvérisation et décalée d'env. 30° par rapport à la verticale, et qu'elle consiste d'une double tuyère concentrique dont la partie intérieure (81) est reliée à une ligne d'amenée pour la solution tampon aqueuse, sa partie enveloppante (83) étant reliée à une ligne d'amenée pour la deuxième suspension.

20. Dispositif selon l'une quelconque des revendications 16 à 19, caractérisé par des aubes (140) courbées en arc de cercle disposées en position verticale sur le rayon extérieur du conduit d'entraînement (154), afin de capter les gouttelettes projetées de la face supérieure (88) du disque de pulvérisation (80) dans le conduit d'entraînement (154) par impact effleurant, et pour les dévier sur la face intérieure du vaisseau en verre (121).

Fig.1

EP 0 310 984 B1

Fig. 2a

Fig. 2

Fig. 3

Fig. 4

Fig. 5

0°          90°          180°          270°          360°

87

89

89

80

87

**Fig. 6**

86

88

82

83

80

81

84

**Fig. 7**